# EUROPEAN PATENT APPLICATION

(11) **EP 1 258 255 A1**
(43) Date of publication of application: **20.11.2002**
(21) Application number: 01112227.2
(22) Date of filing: 18.05.2001
(51) Int. Cl.: A61K 47/48, A61P 35/00

(54) **Conjugates of an antibody to CD44 and a maytansinoid**

(71) Applicant: Boehringer Ingelheim International GmbH, 55218 Ingelheim am Rhein (DE)
(72) Inventor: Adolf, Günther, 1070 Wien (AT); Heider, Karl-Heinz, 2000 Stockerau (AT); Patzelt, Erik, 3002 Purkersdorf (AT); Sproll, Marlies, 82131 Gauting (DE)

(57) **Abstract**

The present invention relates to novel conjugates of antibodies with cytotoxic compounds, pharmaceutical compositions containing such conjugates, and their use in cancer therapy. In particular, the present invention relates to conjugates of antibodies which are specific for CD44 with maytansinods, preferably with N^{2'}-deacetyl-N^{2'}-(3-mercapto-1-oxopropyl)-maytansine. In a particularly preferred embodiment, the antibody/maytansinoid conjugate may be prepared from a maytansinoid of formula 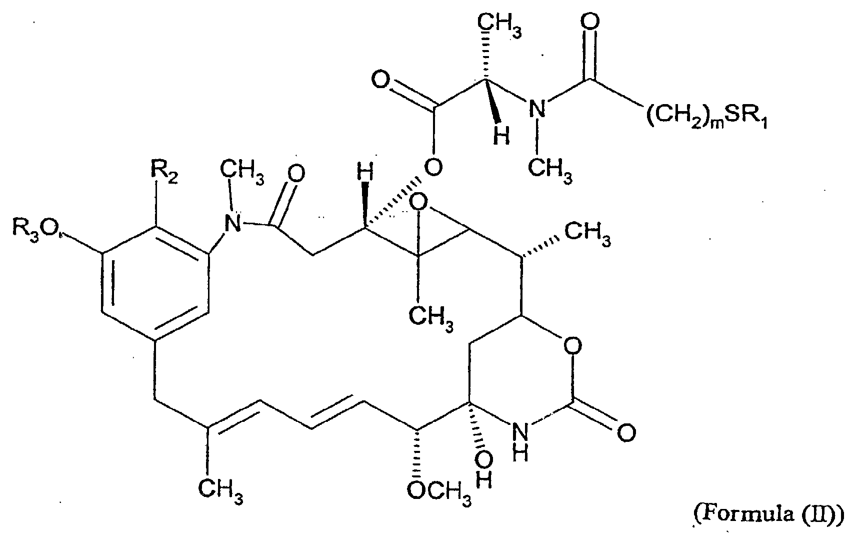 wherein
- R₁: represents H or SR₄, wherein R₄ represents methyl, ethyl, linear alkyl, branched alkyl, cyclic alkyl, simple or substituted aryl, or heterocyclic;
- R₂: represents Cl or H;
- R₃: represents H or CH₃; and
- m: represents 1,2, or 3.
Preferably, R₁ is H or CH₃, R₂ is Cl, R₃ is CH₃, and m=2.
The compound with R₁=H, R₂=Cl, R₃=CH₃, and m=2 is designated DM1 in the literature.

## Description

The invention relates to novel conjugates of antibodies with cytotoxic compounds, pharmaceutical compositions comprising such compounds, and their use in tumor therapy.
There have been numerous attempts to improve the efficacy of antineoplastic drugs by conjugating such drugs to antibodies against tumor-associated antigens in order to elevate local concentration of the drug by targeted delivery to the tumor. Many of these approaches have met limited success, and several reasons have been discussed in the literature to explain the failure. For anticancer drugs acting stoichometrically, like e.g. doxorubicin or methotrexate, relatively high intracellular concentrations are necessary to exert the required cytotoxicity. These concentrations are thought to be difficult to achieve with many antibo-dy-drug conjugates because of (a) insufficient potency of many common anticancer drugs, (b) low cell surface concentration of antigen targets, (c) inefficient internalization of antigen-antibody complexes into the target cell, and (d) inefficient release of free drug from the conjugate inside the target cell (Chari et al., 1992).

Two of the aforementioned drawbacks, namely (a) and (d), have been adressed by the work of Chari and coworkers (Chari et a., 1992; Liu et al., 1996; U.S. Patent No. 5,208,020). They have developed antibody conjugates wherein the antibody is linked to a maytansinoid via a disulfide linkage. Maytansines belong to the class of Ansa macrolide antibiotics, which derive from *Nocardia sp..* The maytansine ansamitocin P-3, produced by bacterial fermentation, is used as a precursor molecule to manufacture maytansinoid DM1. Maytansine and derivatives act as anti-mitotic agents (inhibitors of tubulin polymerization), similar as vincristine, but with markedly higher potency than vincristine or other established chemotherapeutic agents (DM1 is toxic to cells *in vitro* at ∼10⁻¹⁰ M concentration). In contrast to the high cytotoxicity of free maytansinoid, the antibody conjugate has a toxicity which is several orders of magnitude lower on antigen-negative cells compared to antigen-positive cells. The linkage by disulfide bonding has the advantage that these bonds are readily cleaved inside the target cells by intracellular glutathione, releasing highly toxic free drug. This approach has been applied to antibodies against tumor-associated antigens, for example the C242-DM1 conjugate (Liu et al., 1996; Lambert et al., 1998), and HuN901-DM1 (Chari et al., 2000). However, the application of these conjugates is restricted due to the limited expression of the respective target antigens. For example, the antigen recognized by N901 (CD56, N-CAM) is predominanttly expressed by tumors of neuroendocrine origin, the expression of the C242 antigen (CanAg) is mostly limited to tumors derived from the GI tract.
There is, however, still the need to improve this approach by finding suitable tumor-associated antibodies with favorable antigen expression pattern, high and specific cell surface antigen concentration within the target tissue, and efficient internalization process transporting the antigen complexed-antibody conjugate into the cells.

CD44 is a protein which is expressed in several different isoforms on the surface of a wide variety of cell types. The smallest isoform, standard CD44 (CD44s), which is expressed by a variety of different cells, is thought to mediate cell attachment to extracellular matrix components and may transmit a co-stimulus in lymphocyte and monocyte activation. In contrast, expression of splice variants of CD44 which contain the domain v6 (CD44v6) in the extracellular region, is restricted to a subset of epithelia. The physiological role of CD44v6 is not yet fully understood.

CD44v6, as well as other variant exons (CD44v3, CD44v5, CD44v7/v8, CD44v10) has been shown to be a tumor-associated antigen with a favorable expression pattern in human tumors and normal tissues (Heider et al., 1995; Heider et al., 1996; Dall et al., 1996; Beham-Schmid et al., 1998; Tempfer et al., 1998; Wagner et al., 1998) and has been subject to antibody-based diagnostic and therapeutic approaches, in particular radioimmunotherapy (RIT) of tumors (Stromer et al., 2000, WO 95/33771, WO 97/21104).

However, a prerequisite for efficient killing of tumor cells by antibody maytansinoid conjugates is sufficient internalization of the target antigen. Only few data on the internalization of CD44 are available. Bazil et Horejsi reported that downregulation of CD44 on leukocytes upon stimulation with PMA is caused by shedding of the antigen rather than by internalization (Bazil et Horejsi, 1992). Shedding of CD44 is also supported by several reports on soluble CD44 in the serum of tumor patients and normal individuals (Sliutz et al., 1995; Guo et al., 1994; Martin et al., 1997). In a recent paper by Aguiar et al. the amount of internalized CD44 on matrix-intact chondrocytes was determined to be approximately 6% in 4 hours (Aguiar et al., 1999). Similar low levels of internalized CD44v6 on tumor cells were found in experiments performed by BIA (E. Patzelt, unpublished). Taken together, these data suggest that CD44 receptors are more likely subject to shedding than to internalization, and thus CD44 specific antibodies are not to be regarded as suitable candidates for the maytansinoid conjugate approach. This has been supported by in vitro cell proliferation assays wherein Ab_{CD44v6}-DM1 showed only slightly elevated cytotoxicity against antigenpresenting cells as compared to cells lacking the antigen.

It now has been unexpectedly found that CD44 specific antibodies conjugated to highly cytotoxic drugs through a linker which is cleaved under intracellular conditions are very efficient tumor therapeutics in vivo.

The present invention provides novel compounds consisting of a CD44 specific antibody molecule conjugated to a highly cytotoxic drug through a linker which is cleaved under intracellular conditions.

In particular, the present invention provides a compound of formula

**A(LB)**_{**n**} (Formula(I))

wherein
- **A**: is an antibody molecule which is specific for CD44;
- **L**: is a linker moiety;
- **B**: is a compound which is toxic to cells; and
- **n**: is a decimal number with n = 1 to 10

The antibody molecule A has a binding specificity for CD44, preferably variant CD44.

The term "antibody molecule" shall encompass complete immunoglobulins as they are produced by lymphocytes and for example present in blood sera, monoclonal antibodies secreted by hybridoma cell lines, polypeptides produced by recombinant expression in host cells which have the binding specificity of immunoglobulins or monoclonal antibodies, and molecules which have been derived from such immunoglobulins, monoclonal antibodies, or polypeptides by further processing while retaining their binding specificity. In particular, the term "antibody molecule" includes complete immunoglobulins comprising two heavy chains and two light chains, fragments of such immunoglobulins like Fab, Fab', or F(ab)₂ fragments (Kreitman *et al.*, 1993), recombinantly produced polypeptides like chimeric, humanised or fully human antibodies (Breitling et Duebel, 1999; Shin *et al.,* 1989; Güssow *et* Seemann, 1991, Winter *et al.,* 1994, EP 0 239 400, EP 0519596; WO 90/07861 EP 0368684; EP 0438310; WO 9207075, WO 9222653, EP 0 680 040, EP 0 451 216), single chain antibodies (scFv, Johnson *et* Bird, 1991), and the like. Today, antibodies may also be produced without immunising a laboratory animal, e.g. by phage display methods (Aujame et al., 1997). From the aforementioned literature references, the expert knows how to produce these types of antibody molecules, employing state of the art methods like automated peptide and nucleic acid synthesis, laboratory animal immunisation, hybridoma technologies, polymerase chain reaction (PCR), vector and expression technologies, host cell culture, and protein purification methods.

In a preferred embodiment, the antibody molecule of the invention has a binding specificity for the amino acid sequence coded by variant exon v6 of the human CD44 gene. The sequence of variant exon v6 as well as of the other variant exons is known in the art (Screaton *et al.,* 1992; Tölg *et al.*, 1993; Hofmann *et al.*, 1991). A preferred antibody molecule of the invention specifically binds to peptides or polypetides having or containing the amino acid sequence SEQ ID NO: 1 of the accompanying sequence listing, or an allelic variant of said sequence. Preferably, said antibody molecule has binding specificity for an epitope within said sequence. More preferably, the antibody molecule specifically binds to a peptide having the amino acid sequence SEQ ID NO: 2, even more preferably having the amino acid sequence SEQ ID NO: 3. Such antibody molecules may be easily produced with methods known in the art (WO 95/33771, WO 97/21104), e.g. by immunising laboratory animals with chemically synthesised peptides having the aforementioned sequences and proceeding according to methods known in the art (Harlow 1988; Catty 1988).

Preferably, an antibody molecule according to the invention is the murine monoclonal antibody with the designation VFF-18 which is produced by a hybridoma cell line which has been deposited on 07 June 1994 under the accession number DSM ACC2174 with the DSM-Deutsche Sammlung für Mikroorganismen und Zellkulturen GmbH, Mascheroder Weg 1b, D-38124 Braunschweig, Deutschland/Germany. Also preferred are Fab, Fab', or F(ab)₂ fragments of said monoclonal antibody VFF-18. In another preferred embodiment, the antibody molecule is a humanised recombinant antibody, wherein the complementarity determining regions (CDR's) of VFF-18 have been grafted into the respective genes of human immunoglobulin heavy and light chains. In another preferred embodiment, appropriate framework residues of such a CDR-grafted antibody are reverted to murine residues to improve binding affinity. From methods pertinent to the art, the experts knows how to obtain the CDR's of VFF-18, starting with the aforementioned hybridoma with the accession number DSM ACC2174, to choose and obtain appropriate human immunoglobulin genes, to graft the CDR's into these genes, to modify selected framework residues, to express the CDR-grafted antibody in appropriate host cells, e.g. Chinese hamster ovary (CHO) cells, and to test the resulting recombinant antibodies for binding affinity and specificity (see e.g. literature references above). In another preferred embodiment of the invention, the antibody molecule is a recombinant antibody having the CDR's of the antibody VFF-18. Preferably, such a recombinant antibody is a humanised antibody and is a complete immunoglobulin consisting of two complete light and two complete heavy chains. In another preferred embodiment of the invention, the antibody molecule is a recombinant antibody having the same idiotype as the antibody VFF-18. In another preferred embodiment of the invention, the antibody moelcule is a recombinant antibody binding to the same epitope as the antibody VFF-18.

In a particular preferred embodiment, the antibody molecule A is an antibody comprising light chains having the amino acid sequence SEQ ID NO: 4, and heavy chains having the amino acid sequence SEQ ID NO: 6. This antibody is called BIWA4. It is a humanised version of antibody VFF-18 mentioned above, having the complementary determining regions of the murine monoclonal antibody VFF-18 in a completely human framework, and human constant regions. It is therefore an antibody of very low immunogenicity in man, which is a favorable trait. However, as it has no murine framework residues to optimise antigen binding, it has a significanty lower antigen binding affinity as its parent antibody VFF-18, and therefore would not have been regarded as a good candidate for a therapeutic drug. Unexpectedly, it has been found that BIWA4, despite its poor binding affinity, has a very favorable biodistribution and tumor uptake in vivo, making it superior to other humanised versions of VFF-18 with higher binding affintity.

This antibody may be produced as follows. Nucleic acid moelcules coding for the light chain and the heavy chain may be synthesised chemically and enzymatically by standard methods. First, suitable oligonucleotides can be synthesized with methods known in the art (e.g. Gait,M.J., 1984, *Oligonucleotide Synthesis. A Practical Approach.* IRL Press, Oxford, UK), which can be used to produce a synthetic gene. Methods to generate synthetic genes from oligonucleotides are known in the art (e.g. Stemmer et al. 1995, *Single-step assembly of a gene and entire plasmid from large numbers of oligodeoxyribonucleotides*, Gene 164(1): 49-53; Ye et al. 1992, *Gene synthesis and expression in E. coli for pump, a human matrix metalloproteinase*, Biochem Biophys Res Commun 186(1):143-9; Hayden et Mandecki 1988, *Gene synthesis by serial cloning of oligonucleotides,* DNA 7(8): 571-7; Frank et al. 1987, *Methods Enzymol.* 154: 221-249)). Preferably, the nucleic acid molecules encoding the light and heavy chains of BIWA4 have the nucleotide sequences of SEQ ID NO: 5 and SEQ ID NO: 7, respectively. These sequences include sequences coding for leader peptides which are cleaved by the host cell (SEQ ID NO: 5: the first 60 nucleotides; SEQ ID NO: 7: the first 57 nucleotides). Theses nucleic acid molecules encoding the antibody heavy and light chains then may be cloned into an expression vector (either both chains in one vector molecule, or each chain into a separate vector molecule), which then is introduced into a host cell. The host cell preferably is a mamalian host cell, e.g. a COS, CHO, or BHK cell, more preferably a chinese hamster ovary (CHO) cell, e.g. a CHO DG44 (Urlaub and Chasin, Proc. Natl. Acad. Sci. U.S.A. 77(7): 4216-20 (1980)), or CHO-K1 (ATCC CCL-61) cell. The host cell then is cultured in a suitable culture medium under conditions where the antibody is produced, and the antibody is then isolated from the culture according to standard procedures. Procedures for production of antibodies from recombinant DNA in host cells and respective expression vectors are well-known in the art (see e.g. WO 94/11523, WO 97/9351, EP 0481790.)

In order to link the antibody molecule A to the compound B which is toxic to cells, a linking moiety L is used. In the most simple case, the linking moiety L is a chemical bond, preferably a covalent bond which is cleaved under intracellular conditions. In one embodiment of the invention, the bond is between a sulfur atom present in the antibody molecule, e.g. in the side chain of a cystein residue, and another sulfur atom present in the toxic compound. In another embodiment, the linking moiety L consists of one or more atoms or chemical groups. Suitable linking groups are well known in the art and include disulfide groups, thioether groups, acid labile groups, photolabile groups, peptidase labile groups and esterase labile groups. Preferred are disulfide groups and thioether groups.

Conjugates of the antibody molecules of the invention and toxic compound can be formed using any techniques presently known or later developed. The toxic compound can be modified to yield a free amino group and then linked to the antibody molecule via an acidlabile linker, or a photolabile linker. The toxic compound can be condensed with a peptide and subsequently linked to an antibody molecule to produce a peptidase-labile linker. The toxic compound can be treated to yield a primary hydroxyl group, which can be succinylated and linked to an antibody molecule to produce a conjugate that can be cleaved by intracellular esterases to liberate free drug. Most preferably, the toxic compound is treated to create a free or protected thiol group, and then one or many disulfide or thiol-containing toxic compounds are covalently linked to the antibody molecule via disulfide bond(s).

For example, antibody molecules can be modified with crosslinking reagents such as N-succinimidyl 3-(2-pyridyldithio)propionate (SPDP), 4-succinimidyl-oxycarbonyl-α-methylα-(2-pyridyldithio)-toluene (SMPT), N-succinimidyl-3-(2-pyridyldithio)-butyrate (SDPB), 2-iminothiolane, or acetylsuccinic anhydride by known methods. See, Carlsson et al, 1978; Blattler et al 1985; Lambert et al, 1983; Klotz et al, 1962; Liu et al, 1979; Blakey and Thorpe, 1988; Worrell et al, 1986. The antibody molecule containing free or protected thiol groups thus derived is then reacted with a disulfide- or thiol-containing toxic compound to produce conjugates. The conjugates can be purified by HPLC or by gel filtration.

Toxic compounds used for coupling may act either cytostatic or cytotoxic and lead to cell cycle arrest or cell death. These compounds may act at different stages during the cell cycle, e.g. by interference with nucleic acid synthesis, inactivation of nucleic acids, or by binding to tubulin.

In a preferred embodiment, the compound B which is toxic to cells is a maytansinoid. Maytansinoids suitable for conjugating to antibodies for use in cancer therapy, including preparation of said maytansinoids and their linkage to antibody molecules, have been described by Chari and Coworkers (Chari et al., 1992; Liu et al., 1996; U.S. Patent No. 5,208,020). These maytansinoids may be used for the present invention. In a preferred embodiment, the toxic compound is N^{2'}-deacetyl-N^{2'}-(3-mercapto-1-oxopropyl)-Maytansine (CAS Number 139504-50-0), also referred to as DM1. Preferably, said maytansinoid is a maytansinol derivative linked to the antibody molecule via a disulfide bridge at the C-3 position of maytansinol. In a particularly preferred embodiment, the antibody/maytansinoid conjugate may be prepared from a maytansinoid of formula 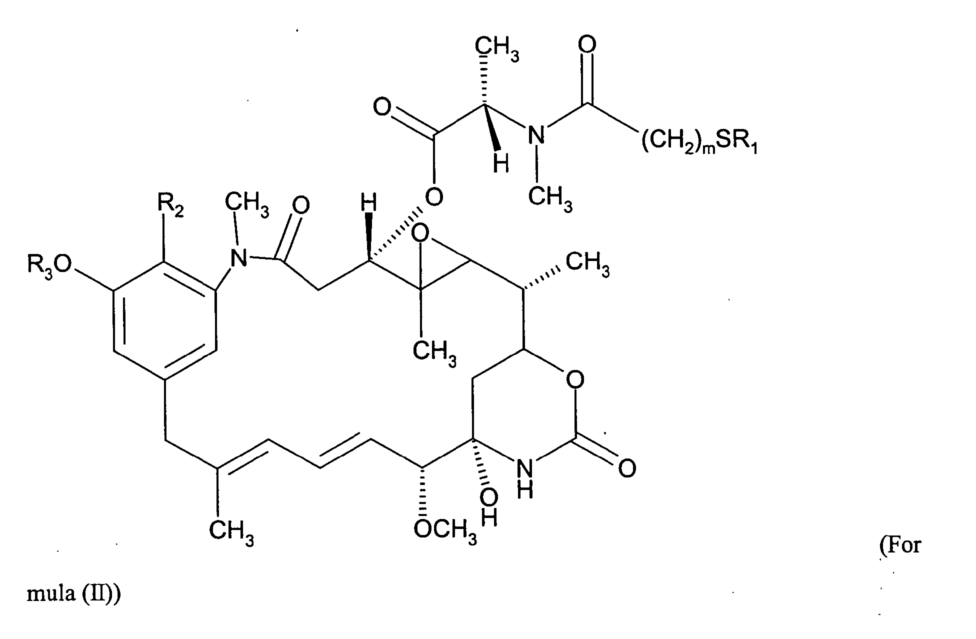 wherein
R₁ represents H or SR₄, wherein R₄ represents methyl, ethyl, linear alkyl, branched alkyl, cyclic alkyl, simple or substituted aryl, or heterocyclic;
R₂ represents Cl or H;
R₃ represents H or CH₃; and
m represents 1, 2, or 3.

Preferably, R₁ is H or CH₃, R₂ is Cl, R₃ is CH₃, and m = 2.

The compound with R₁ = H, R₂ = Cl, R₃ = CH₃, and m = 2 is designated DM1 in the literature.

In a preferred embodiment, the compund of the invention has the formula 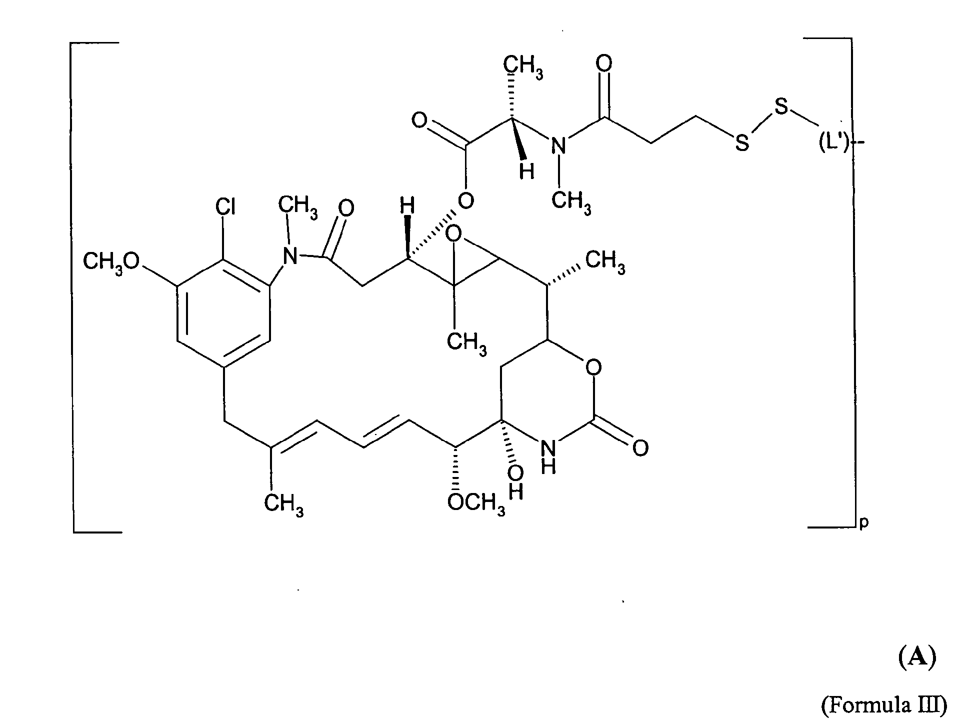 wherein
**A** is an antibody molecule which is specific for CD44, preferably specific for the variant exon v6, preferably specific for the amino acid sequence SEQ ID NO: 3;
(L') is an optional linker moiety
p is a decimal number with p = 1 to 10

Preferably, p is 3 to 4, more preferably about 3.5.

Methods for preparing such maytansinoids are known in the art (see in particular US 5,208,020, Example 1).

Upon intracellular cleavage, the free toxic compound is released. The free drug released from the compound A(LB)ₙ may have the formula B-X, wherein X is an atom or a chemical group, depending on the nature of the cleaving reaction. Preferably, X is a hydrogen atom, as for example when the linker moiety is just a covalent bond between two sulfur atoms, or a hydroxyl group. The cleavage site may also be within the linker moiety if the linker moiety is a chemical group, generating free drug of formula B-L"-X upon cleavage, wherein X is an atom or a chemical group, depending on the nature of the cleaving reaction. Preferably, X is a hydrogen atom or a hydroxyl group.

In a preferred embodiment, the compound of formula (I) is less toxic than the toxic compound B, B-X or B-L"-X released upon intracellular cleavage. Methods of testing cytotoxicity in vitro are known in the art (Goldmacher et al., 1985; Goldmacher et al., 1986; see also US 5,208,020, Example 2). Preferably, the compound (I) is 10 times or more, more preferably 100 times or more, or even 1000 times or more less toxic than the free drug released upon cleavage.

Preferably, antibody molecule/maytansinoid conjugates are those that are joined via a disulfide bond, as discussed above, that are capable of delivering maytansinoid molecules. Such cell binding conjugates are prepared by known methods such as modifying monoclonal antibodies with succinimidyl pyridyl-dithiopropionate (SPDP) (Carlsson et al, 1978). The resulting thiopyridyl group is then displaced by treatment with thiol-containing maytansinoids to produce disulfide linked conjugates. Alternatively, in the case of the aryldithiomaytansinoids, the formation of the antibody conjugate is effected by direct displacement of the aryl-thiol of the maytansinoid by sulfhydryl groups previously introduced into antibody molecules. Conjugates containing 1 to 10 maytansinoid drugs linked via a disulfide bridge are readily prepared by either method. In this context, it is understood that the decimal number n in the formula A(LB)ₙ is an average number as not all conjugate molecules of a given preparation may have the identical integer of LB residues attached to the antibody molecule.

More specifically, a solution of the dithiopyridyl modified antibody at a concentration of 1 mg/ml in 0.1M potassium phosphate buffer, at pH 7.0 containing 1 mM EDTA is treated with the thiol-containing maytansinoid (1.25 molar equivalent/dithiopyridyl group). The release of pyridine-2-thione from the modified antibody is monitored spectrophotometrically at 343nm and is complete in about 30 min. The antibody-maytansinoid conjugate is purified and freed of unreacted drug and other low molecular weight material by gel filtration through a column of Sephadex G-25. The number of maytansinoids bound per antibody molecule can be determined by measuring the ratio of the absorbance at 252 nm and 280 nm. An average of 1-10 maytansinoid molecules/antibody molecule can be linked via disulfide bonds by this method.

In a further embodiment, the present invention relates to a method of production of a compound of formula (I) comprising the steps:
(a) introducing free or protected thiol groups into an antibody molecule which is specific for CD44;
(b) reacting the antibody molecule of step (a) with a compound which is toxic to cells, said compound having one or more disulfide or thiol groups; and
(c) recovering the resulting conjugate.

Preferably, the antibody molecule is specific for the amino acid sequence SEQ ID NO: 3, and the compound which is toxic to cells is of formula (II), preferably with R₁ = H, R₂ = Cl, R₃ = CH₃, and m = 2.

In a further embodiment, the present invention relates to a pharmaceutical composition comprising a compound of formula (I), preferably together with a pharmaceutically acceptable carrier, excipient, or diluent.

Suitable pharmaceutically acceptable carriers, diluents, and excipients are well known and can be determined by those of skill in the art as the clinical situation warrants. Examples of suitable carriers, diluents and/or excipients include: (1) Dulbecco's phosphate buffered saline, pH about 7.4, containing about 1 mg/ml to 25 mg/ml human serum albumin, (2) 0.9% saline (0.9% w/v NaCl), and (3) 5% (w/v) dextrose.

More preferably, the antibody molecule present in the pharmaceutical composition is the monoclonal antibody VFF-18, or a recombinant antibody having the CDR's of the antibody VFF-18, preferably in a human framework. Preferably, the toxic compound is the maytansinoid of formula (II).

The conjugate may for example be clinically used ex vivo to remove tumor cells from bone marrow prior to autologous transplantation in cancer treatment. Treatment can be carried out as follows. Bone marrow is harvested from the patient or other individual and then incubated in medium containing serum to which is added the compound of formula (I) according to the invention, concentrations range from about 10 µM to 1 pM, for about 30 minutes to about 48 hours at about 37°C. The exact conditions of concentration and time of incubation (=dose) are readily determined by the skilled artisan. After incubation, the bone marrow cells are washed with medium containing serum and returned to the patient by i.v. infusion according to known methods. In circumstances where the patient receives other treatment such as a course of ablative chemotherapy or total-body irradiation between the time of harvest of the marrow and reinfusion of the treated cells, the treated marrow cells are stored frozen in liquid nitrogen using standard medical equipment.

In a further embodiment, the present invention relates to a method of treatment of cancer comprising applying a pharmaceutical composition as described before to a patient. Preferably, the cancer is head and neck squamous cell carcinoma (SCC), esophagus SCC, lung SCC, skin SCC, breast adenocarcinoma (AC), lung AC, cervix SCC, pancreas AC, colon AC, or stomach AC.

For clinical treatment of cancer, the compound of formula (I) according to the invention will be supplied as solutions that are tested for sterility and for endotoxin levels. Examples of suitable protocols of conjugate administration are as follows. Conjugates may be given weekly for 1 to 6 weeks either as an i.v. bolus , or as a continuous infusion for 5 days. Bolus doses can be given in 50 to 100 ml of normal saline to which 5 to 10 ml of human serum albumin has been added. Continuous infusions can be given in 250 to 500 ml of normal saline, to which 25 to 50 ml of human serum albumin has been added, per 24 hour period. Dosages will be 10 mg to 400 mg/m² of body surface area per application. The dose applied to the patient per administration has to be high enough to be effective, but must be below the dose limiting toxicity (DLT). In general, a sufficiently well tolerated dose below DLT will be considered maximum tolerated dose (MTD). The expert knows how to determine the MTD (Lambert et al., 1998). For weekly administrations, the MTD can be expected to be in the range of 100 to 200 mg/m². Alternatively, intervals between applications may be longer, e.g. two to four weeks, preferably three weeks. In this case, the MTD can be expected to be in the range of 200 to 300 mg/m². Alternatively, application may be in 5 daily doses, followed by a break of several weeks after which treatment may be repeated. In this case, the MTD per administration can be expected to be lower than 100 mg/m². For example, conjugates can be administered as a single i.v. infusion with a rate of 3 mg/min every 21 days. Up to 7 cycles of treatment were applied.

Dose, route of administration, application scheme, repetition and duration of treatment will in general depend on the nature of the disease (type, grade, and stage of the tumor etc.) and the patient (constitution, age, gender etc.), and will be determined by the medical expert responsible for the treatment. Besides treatment of solid tumors, therapeutic application according to the invention may be particularly advantageous as an adjuvant to surgical intervention, to treat minimal residual disease.

In a further embodiment, the invention relates to the use of a compound of formula (I) for the preparation of a pharmaceutical composition for the treatment of cancer. More preferably, the antibody molecule present in the pharmaceutical composition is the monoclonal antibody VFF-18, or a recombinant antibody having the CDR's of the antibody VFF-18, preferably in a human framework. Preferably, the toxic compound has the formula (II). Preferably, the cancer is head and neck squamous cell carcinoma (SCC), esophagus SCC, lung SCC, skin SCC, breast adenocarcinoma (AC), lung AC, cervix SCC, pancreas AC, colon AC, or stomach AC.

### References

Aguiar DJ, Knudson W, and Knudson CB. Internalization of the hyaluronan receptor CD44 by chondrocytes. Exp.Cell.Res. 252: 292-302, 1999.
Aujame L, Geoffroy F, Sodoyer R. High affinity human antibodies by phage display. *Hum Antibodies* 1997;8(4):155-68
Bazil V and Horejsi V. Shedding of the CD44 adhesion molecule from leukocytes induced by anti-CD44 monoclonal antibody simulating the effect of a natural receptor ligand. J Immunol 149 (3):747-753, 1992.
Blattler et al, Biochem. 24: 1517-1524 (1985).
Breitling F, Duebel S: Recombinant Antibodies. John Wiley, New York 1999.
Carlsson et al, Biochem. J. 173: 723-737 (1978).
Catty D. Antibodies. Oxford IR Press 1988.
Chari RVJ, Martell BA, Gross JL, Cook SB, Shah SA, Blättler WA, McKenzie SJ, Goldmacher VS. Immunoconjugates containing novel maytansinoids: promising anticancer drugs. Cancer Research 52: 127-31, 1992.
Chari RVJ, Derr SM, Steeves RM, Widdison WC: Dose-response of the anti-tumor effect of HUN901-DM1 against human small cell lung cancer xenografts. Proceedings of the American Association of Cancer Research (2000) 41(April 1-5) Abs 4405.
Goldmacher et al., J Immunol 135: 3648-3651, 1985.
Goldmacher et al., J Cell Biol 102: 1312-1319, 1986.
Güssow D, Seemann G. Humanization of monoclonal antibodies. *Methods Enzymol. 203:* 99-121 (1991)
Guo YJ, Liu G, Wang X, Jin D, Wu M, Ma J, and Sy MS. Potential use of soluble CD44 in serum as indicator of tumor burden and metastasis in patients with gastric or colon cancer. Cancer Res 54 (2): 422-426, 1994.
Harlow L D. Antibodies. Cold Spring Harbor Lab.1988
Heider KH, Mulder JWR, Ostermann E,Susani S, Patzelt E, Pals ST, Adolf GRA. Splice variants of the cell surface glycoprotein CD44 associated with metastatic tumor cells are expressed in normal tissues of humans and cynomolgus monkeys. Eur. J. Cancer 31A: 2385-2391, 1995.
Heider KH, Sproll M, Susani S, Patzelt E, Beaumier P, Ostermann O, Ahorn H, Adolf GRA. Characterization of a high affinity monoclonal antibody antibody specific for CD44v6 as candidate for immunotherapy of squamous cell carcinomas. Cancer Immunology Immunotherapy 43: 245-253, 1996.
Hofinann, M., Rudy, W., Zöller, M., Tölg, C., Ponta, H., Herrlich P., and Günthert, U. CD44 splice variants confer metastatic behavior in rats: homologous sequences are expressed in human tumor cell lines. *Cancer Res. 51:* 5292-5297 (1991).
Johnson S, Bird R E. Construction of single-chain derivatives of monoclonal antibodies and their production in *Escherichia coli. Methods Enzymol. 203:* 88-98 (1991).
Klotz et al, Arch. Biochem. Biophys. 96: 605 (1962)
Kreitman R J Hansen H J, Jones A L, FitzGerald D J P, Goldenberg D M, Pastan I. *Pseudomonas* exotoxin-based immunotoxins containing the antibody LL2 or LL2-Fab' induce regression of subcutaneous human B-cell lymphoma in mice. *Cancer Res. 53:* 819-825 (1993).
Lambert et al, Biochem. 22: 3913-3920 (1983).
Lambert JM, Derr SM, Cook S, Braman G, Widdison W, Chari RVJ. Pharmacokinetics, in vivo stability, and toxicity of the Tumor-activated prodrug, C242-DM1, a novel colorectal cancer agent. Proceedings of the American Association of Cancer Research (1998) 39: Abs 3550
Liu et al, Biochem. 18: 690 (1979), Blakey and Thorpe, Antibody, Immunoconjugates and Radiopharmaceuticals, 1: 1-16 (1988).
Liu C, Tadayoni BM, Bourret LA, Mattocks KM, Derr SM, Widdison WC, Kedersha NL, Ariniello PD, Goldmacher VS, Lambert JM, Blättler WA, Chari RVJ. Eradication of large colon tumor xenografts by targeted delivery of maytansinoids. Proc Natl Acad Sci USA 93: 8618-23, 1996.
Martin S, Jansen F, Bokelmann J, and Kolb H. Soluble CD44 splice variants in metastasizing human breast cancer. Int J Cancer 74 (4): 443-445, 1997.
Screaton, G.R., Bell, M.V., Jackson, D.G., Cornelis, F.B., Gerth, U., and Bell, J. I. Genomic structure of DNA encoding the lymphocyte homing receptor CD44 reveals at least 12 alternatively spliced exons. *Proc. Natl. Acad. Sci. U.S.A. 89:* 12160-12164 (1992).
Shin S-U, Morrison S L. Production and properties of chimeric antibody molecules. *Methods Enzymol. 178:* 459-476 (1989).
Sliutz G, Tempfer C, Winkler S, Kohlberger P, Reinthaller A, and Kainz C. Immunohistochemical and serological evaluation of CD44 splice variants in human ovarian cancer. Br.J.Cancer 72: 1494-1497, 1995.
Stroomer JW, Roos JC, Sproll M, Quak JJ, Heider KH, Wilhelm BJ, Castelijns JA, Meyer R, Kwakkelstein MO, Snow GB, Adolf GR, van Dongen GA. Safety and biodistribution of 99mTechnetium-labeled anti-CD44v6 monoclonal antibody BIWA 1 in head and neck cancer patients. Clin Cancer Res 6 (8):3046-55, 2000
Tölg, C., Hofmann, M., Herrlich, P., and Ponta, H. Splicing choice from ten variant exons establishes CD44 variability. *Nucleic Acids. Res. 21:* 1225-1229 (1993).
Tolcher AW et al. SB-408075, a maytansinoid immunoconjugate directed to the C242 antigen: a phase I pharmacokinetic and biologic correlative study. Poster presented at 11^{th} Symp. on new drugs in cancer therapy (Nov 7-10, 2000 in Amsterdam).
Winter, G., Griffith, A. D., Hawkins, R. E., Hoogenboom, H. R. Making antibodies by phage display technology. *Ann. Rev. Immunol. 12,* 433-455 (1994).
Worrell et al, Anti-Cancer Drug Design 1:179-184(1986).

### Examples

### 1. Material and Methods

### 1.1. In vitro cell proliferation assay

For determination of viable cells the Cell Titer 96® AQᵤₑₒᵤₛ non-radioactive cell proliferation assay (Promega) was used. 5000 cells per well were seeded into 96-well plates in 90 µl medium without phenole red. Cells were allowed to settle down for 1 to 3 h and then serial dilutions of the immunoconjugate in 10 µl PBS were added. Cells without immunoconjugate served as negative control. Cells were incubated for 4 days at 37°C in a humified 5% CO₂ atmosphere and then 20 µl MTS/ PMS were added according to the manufacturer's recommendation. After additional 1 to 4 h incubation at 37°C the absorbance at 490 nm was recorded using an ELISA plate reader. For each cell line triplicates were analyzed. The percentage of the surviving cell fraction and the IC50 value were calculated using the GraphPad Prism® (Version 3.0) software.

### 1.2. Efficacy studies in nude mice

A humanised recombinant antibody BIWA4 as described above was linked to the maytansinoid DM1 as described above. The conjugate was designated BIWI 1.

*In vivo* anti-tumor efficacy of BIWI 1 was tested in two nude mouse xenograft models applying antigen-positive human tumors, which differed in tumor origin, extent and homogeneity of CD44v6 expression: A431 (ATCC # CRL 1555; epidermoid carcinoma of the vulva), FaDu (ATCC # HTB 43; squamous cell carcinoma of the pharynx). Tumor cell lines A431 and FaDu were received from ATCC and cultured in RPMI1640 medium containing 10% fetal calf serum and supplements. 1x10⁶ tumors cells were transplanted subcutaneously into the right flank of 6 week old female NMRI-nu/nu mice. Treatment started when the tumors reached an average size of 102 to 185 mm³. Treatment consisted of i.v. injections of BIWI 1 given on five consecutive days, starting at day 1. 3 different doses of BIWI 1 were tested in parallel: 2.1 mg/kg/d BIWI 1 corresponding to 30 µg/kg/d DM1, 7 mg/kg/d BIWI 1 corresponding to 100 µg/kg/d DM1, and 21 mg/kg/d BIWI 1 corresponding to 300 µg/kg/d DM1. Control animals were either untreated (PBS) or treated with unconjugated antibody (control antibody, 21 mg/kg/d). Tumor growth was monitored by measuring tumor size. A tumor response was rated as complete response when the tumor completely disappeared at any time after start of treatment. The response was rated as partial response when the tumor volume decreased after treatment but thereafter started regrowing. The tolerability of the treatment was monitored by measuring mouse weight during the whole observation period.

### 2. Results and Discussion

### 2.1. In vitro cytotoxicity of BIWI 1

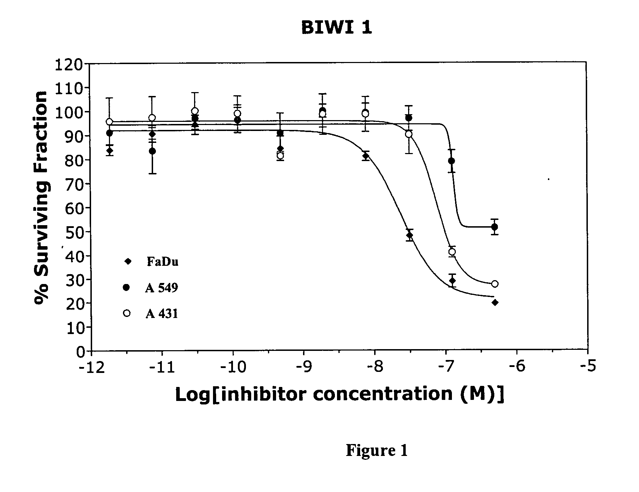

The *in vitro* cytotoxicity of BIWI 1 was evaluated using the antigen-positive cell lines A431 and FaDu, and the antigen-negative cell line A459. Cells were exposed to different concentrations of BIWI 1 for 4 days, then stained with MTS/ PMS and assayed on an ELISA plate reader. The surviving fractions of cells were then calculated using the GraphPad Prism® software package. The results are shown in Figure 1. BIWI 1 was effective in killing the antigen-positive A431 cells with an IC₅₀ of about 7.6 x 10⁻⁸ M and the second antigen-positive cell line, FaDu, with an IC₅₀ of about 2.4 x 10⁻⁸ M. The antigen-negative cell line, A549, was effected by the conjugate with an IC₅₀ of about 1.3 x 10⁻⁷ M with a surviving fraction of 50% at the highest concentration of BIWI 1 tested (5 x 10⁻⁷ M). These results show that BIWI 1 is only slightly more cytotoxic against antigen-positive cells than antigen-negative cells *in vitro*.

### 2.2. Efficacy in A431 xenografted nude mice

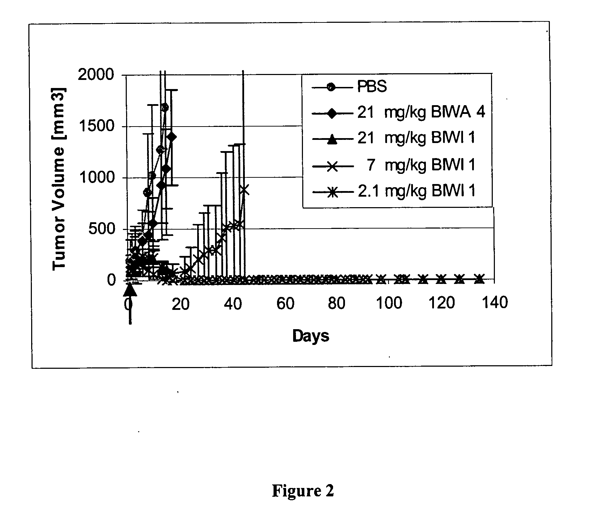

Groups of 5 mice were treated with 2.1 mg/kg/d BIWI 1, 7 mg/kg/d BIWI 1, 21 mg/kg/d BIWI 1, and 21 mg/kg/d control antibody, respectively. The average tumor size at start of treatment was 185 +/- 217 mm³ (PBS), 133 +/- 115 mm³ (control antibody), 107 +/- 63 mm³ (21 mg/kg/d BIWI 1), 132 +/- 73 mm³ (7 mg/kg/d BIWI 1), and 107 +/- 63 mm³ (2.1 mg/kg/d BIWI 1), respectively. The average tumor volume of each group during the observation period is shown in Figure 2. Tumors treated with control antibody showed similar growth as untreated tumors, the tumor volume doubling time was approximately 5 days. In animals treated either with 7 mg/kg/d BIWI 1 or 21 mg/kg/d BIWI 1 all tumors responded completely and disappeared around day 17. No tumor regrowth was observed until the end of the observation period (day 134). Tumors treated with 2.1 mg/kg/d responded completely in 3/5 cases with no tumor regrowth until day 134. The remaining 2 tumors showed a partial response but ultimately regrew. These results show that BIWI 1 induces a dose-dependent anti-tumor response in A431 xenografted nude mice, with complete and long-lasting responses from 2.1 mg/kg/d BIWI 1 to 21 mg/kg/d BIWI 1. Unconjugated control antibody shows no anti-tumor effect.

See figure 1.

### 2.2. Efficacy in FaDu xenografted nude mice

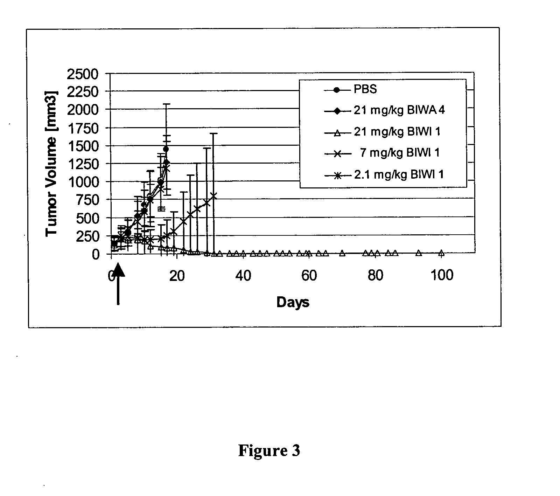

Groups of 6 mice were treated with 2.1 mg/kg/d BIWI 1, 7 mg/kg/d BIWI 1, 21 mg/kg/d BIWI 1, and 21 mg/kg/d control antibody, respectively. The average tumor size at start of treatment was 142 +/- 82 mm³ (PBS), 134 +/- 86 mm³ (control antibody), 129 +/- 74 mm³ (21 mg/kg/d BIWI 1), 132 +/- 97 mm³ (7 mg/kg/d BIWI 1), and 149 +/- 96 mm³ (2.1 mg/kg/d BIWI 1), respectively. The average tumor volume of each group during the observation period is shown in Figure 3. Tumors treated with control antibody and 2.1 mg/kg/d BIWI 1 showed similar growth as untreated tumors, the tumor volume doubling time was approximately 5 days. In animals treated with 21 mg/kg/d BIWI 1 all tumors responded completely and disappeared around day 24. No tumor regrowth was observed until the end of the observation period (day 107). 1/6 tumors treated with 7 mg/kg/d BIWI 1 responded completely, 3/6 tumors showed partial responses. The remaining 2 tumors grew similar to untreated control tumors. These results show that BIWI 1 induces a dose-dependent anti-tumor response in FaDu xenografted nude mice, with complete and long-lasting responses from 7 mg/kg/d BIWI 1 to 21 mg/kg/d BIWI 1. Unconjugated control antibody shows no anti-tumor effect.

See figure 2.

### 2.4. Tolerability in nude mice

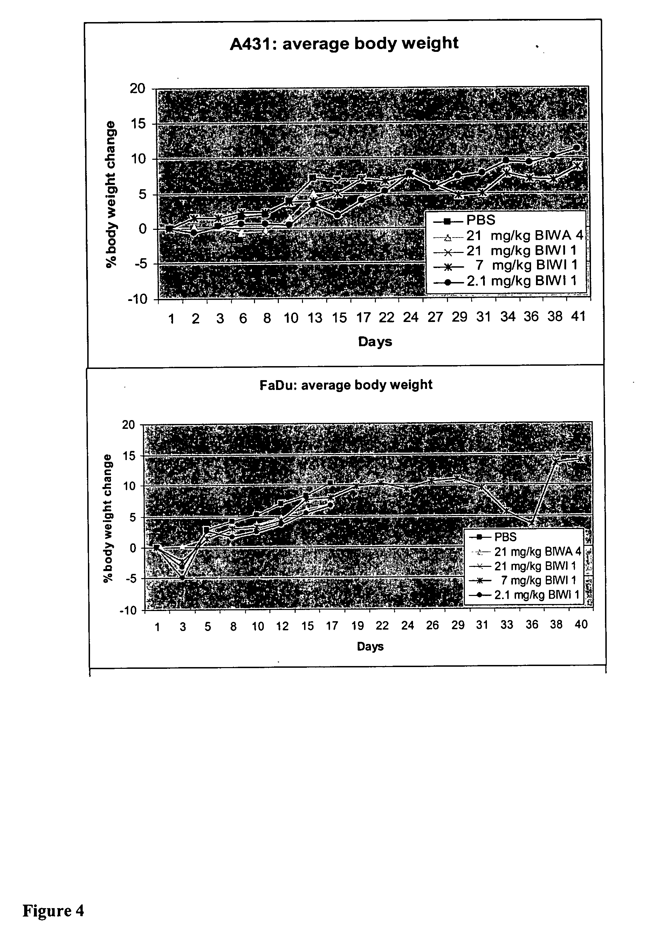

The tolerability of BIWI 1 treatment was determined by monitoring mouse weight during the whole duration of the experiment in the 2 models. The maximum observed average weight loss per group was 5% in FaDu xenografted mice treated with 21 mg/kg/d BIWI 1 (Figure 4). The weight loss started around day 3 of treatment and lasted until day 10, thereafter animals regained weight and behaved similar as control animals. In all other dose groups weight loss was similar to vehicle control (PBS). An average weight loss of 5% or less in all treatment groups indicates good tolerability of BIWI 1 treatment at the given doses in nude mice. As BIWI 1 does not cross-react with mouse CD44v6, only antigenindependent effects such as toxicity caused by free DM1 can be monitored in this experiment.

### Figures

**Figure 1:** in vitro cytotoxicity of BIWI 1. The antigen-positive cell lines A431 and FaDu and the antigen-negative cell line A549 were used.
**Figure 2:** Efficacy of BIWI 1 treatment in nude mice xenografted with A431 tumors. The average tumor volumes per group with standard deviations are shown, the treatment groups are indicated. The arrow indicates start of treatment (day 1).
**Figure 3:** Efficacy of BIWI 1 treatment in nude mice xenografted with FaDu tumors. The average tumor volumes per group with standard deviations are shown, the treatment groups are indicated. The arrow indicates start of treatment (day 1).
**Figure 4:** Tolerability of BIWI 1 treatment. The average body weight change of all treatment groups in the 2 investigated models is shown. Day 1: start of treatment.

## Claims

1. A compound of formula
**A(LB)**_{**n**}
wherein
**A** is an antibody molecule which is specific for CD44;
**L** is a linker moiety;
**B** is a compound which is toxic to cells; and
**n** is an decimal number with n = 1 to 10

2. The compound of claim 1 wherein said linker moiety has a chemical bond capable of being cleaved inside a cell.

3. The compound of claim 2 wherein said chemical bond is a disulfide bond.

4. The compound of claims 1 to 3, wherein the antibody molecule is specific for the exon v6 of human CD44.

5. The compound of claims 1 to 4, wherein the antibody molecule is specific for an epitope within the amino acid sequence SEQ ID NO: 3.

6. The compound of claims 1 to 5, wherein the antibody molecule is the monoclonal antibody VFF-18 (DSM ACC2174) or a recombinant antibody having the complementary determining regions (CDRs) of VFF-18.

7. The compound of claims 1 to 6, wherein the antibody molecule comprises light chains having the amino acid sequence SEQ ID NO: 4, and heavy chains having the amino acid sequence SEQ ID NO: 6.

8. The compound of claims 1 to 7, wherein the toxic compound **B** is a maytansinoid.

9. The compound of claim 8 wherein the maytansinoid has the formula 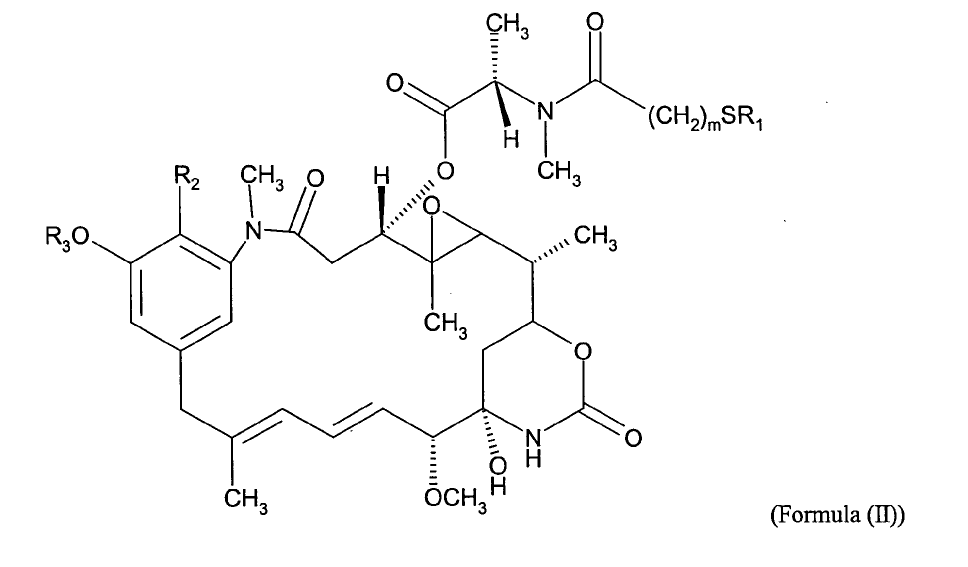 wherein
R₁ represents H or SR₄, wherein R₄ represents methyl, ethyl, linear alkyl, branched alkyl, cyclic alkyl, simple or substituted aryl, or heterocyclic;
R₂ represents Cl or H;
R₃ represents H or CH₃; and
m represents 1, 2, or 3.

10. The compound of claim 9, wherein R₁ is H or CH₃, R₂ is Cl, R₃ is CH₃, and m = 2

11. The compound of claims 1 to 10 of formula 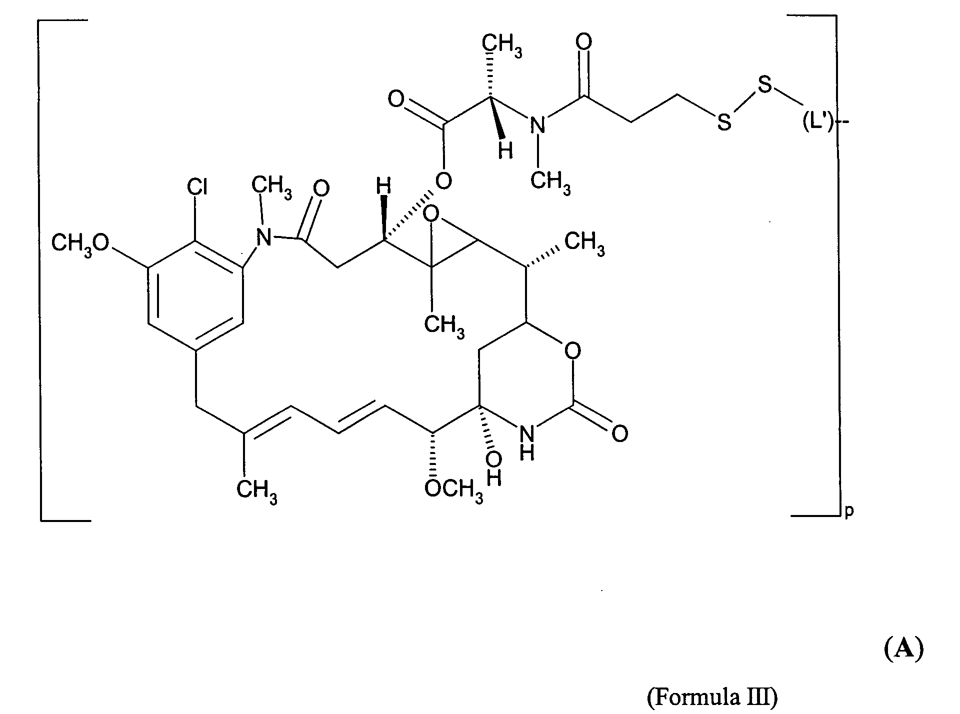 wherein
**A** is an antibody molecule which is specific for CD44,
(L') is an optional linker moiety
p is a decimal number with p = 1 to 10

12. The compound of claims 1 to 11 wherein p is 3 to 4.

13. Method of production of a compound according to claims 1 to 11 comprising the steps:
(a) introducing one ore more free or protected thiol groups into an antibody molecule which is specific for CD44;
(b) reacting the antibody molecule of step (a) with a compound which is toxic to cells, said compound having one or more disulfide or thiol groups; and
(c) recovering the resulting conjugate.

14. Pharmaceutical composition comprising a compound according to claims 1 to 12 and a pharmaceutically acceptable carrier, diluent, or excipient.

15. Use of a compound according to claims 1 to 14 for the preparation of a pharmaceutical composition for the treatment of cancer.

16. Use of a compound according to claims 1 to 11 for the treatment of cancer.


SEQUENCE LISTING

<110> Boehringer Ingelheim International GmbH

<120> Cytotoxic CD44 Antibody Immunoconjugates

<130>

<140>
<141>

<160> 7

<170> PatentIn Ver. 2.1

<210> 1
<211> 42
<212> PRT
<213> Homo sapiens

<223> Description of Artificial Sequence: SEQ ID NO: 1

<400> 1
Gln Ala Thr Pro Ser Ser Thr Thr Glu Glu Thr Ala Thr Gln Lys Glu
  1               5                  10                  15

Gln Trp Phe Gly Asn Arg Trp His Glu Gly Tyr Arg Gln Thr Pro Arg
             20                  25                  30

Glu Asp Ser His Ser Thr Thr Gly Thr Ala
         35                  40

<210> 2
<211> 14
<212> PRT
<213> Homo sapiens

<223> Description of Artificial Sequence: SEQ ID NO: 2

<400> 2
Gln Trp Phe Gly Asn Arg Trp His Glu Gly Tyr Arg Gln Thr
  1               5                  10

<210> 3
<211> 11
<212> PRT
<213> Homo sapiens

<223> Description of Artificial Sequence: SEQ ID NO: 3

<400> 3
Trp Phe Gly Asn Arg Trp His Glu Gly Tyr Arg
  1               5                  10

<210> 4
<211> 213
<212> PRT

<213> Artificial Sequence

<220>
<223> Description of Artificial Sequence: Humanised
      Murine Antibody BIWA4 Light Chain SEQ ID NO: 4

<400> 4
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Ile Asn Tyr Ile
            20                  25                  30

Tyr Trp Tyr Gln Gln Lys Pro Gly Gln Ala Pro Arg Leu Leu Ile Tyr
        35                  40                  45

Leu Thr Ser Asn Leu Ala Ser Gly Val Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Leu Gln Trp Ser Ser Asn Pro Leu Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210


<210> 5
<211> 702
<212> DNA
<213> Artificial Sequence

<220>
<223> Description of Artificial Sequence: Humanised
      Murine Antibody BIWA4 Light Chain SEQ ID NO: 5

<400> 5

atggaagccc cagctcagct tctcttcctc ctgctgctct ggctcccaga taccaccgga 60
gaaattgttc tcacccagtc tccagcaacc ctgtctctgt ctccagggga gagggccacc 120
ctgtcctgca gtgccagctc aagtataaat tacatatact ggtaccagca gaagccagga 180
caggctccta gactcttgat ttatctcaca tccaacctgg cttctggagt ccctgcgcgc 240
ttcagtggca gtgggtctgg aaccgacttc actctcacaa tcagcagcct ggagcctgaa 300
gattttgccg tttattactg cctgcagtgg agtagtaacc cgctcacatt cggtggtggg 360
accaaggtgg agattaaacg tacggtggct gcaccatctg tcttcatctt cccgccatct 420
gatgagcagt tgaaatctgg aactgcctct gttgtgtgcc tgctgaataa cttctatccc 480
agagaggcca aagtacagtg gaaggtggat aacgccctcc aatcgggtaa ctcccaggag 540
agtgtcacag agcaggacag caaggacagc acctacagcc tcagcagcac cctgacgctg 600
agcaaagcag actacgagaa acacaaagtc tacgcctgcg aagtcaccca tcagggcctg 660
agctcgcccg tcacaaagag cttcaacagg ggagagtgtt ga 702

<210> 6
<211> 444
<212> PRT
<213> Artificial Sequence

<220>
<223> Description of Artificial Sequence: Humanised
      Murine Antibody BIWA4 Heavy Chain SEQ ID NO: 6

<400> 6
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Thr Ile Ser Ser Gly Gly Ser Tyr Thr Tyr Tyr Leu Asp Ser Ile
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gln Gly Leu Asp Tyr Trp Gly Arg Gly Thr Leu Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val Glu Pro Lys Ser Cys Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
        435                 440

<210> 7
<211> 1392
<212> DNA
<213> Artificial Sequence

<220>
<223> Description of Artificial Sequence: Humanised
      Murine Antibody BIWA4 Heavy Chain SEQ ID NO: 7

<400> 7
atggagtttg ggctgagctg gctttttctt gtggctattt taaaaggtgt ccagtgtgaa 60

gtgcagctgg tggagtctgg gggaggctta gtgaagcctg gagggtccct aagactctcc 120
tgtgcagcct ctggattcac tttcagtagc tatgacatgt cttgggttcg ccaggctccg 180
gggaaggggc tggagtgggt ctcaaccatt agtagtggtg gtagttacac ctactatcta 240
gacagtataa agggccgatt caccatctcc agagacaatg ccaagaactc cctgtacctg 300
caaatgaaca gtctgagggc tgaggacacg gccgtgtatt actgtgcaag acaggggttg 360
gactactggg gtcgaggaac cttagtcacc gtctcctcag ctagcaccaa gggcccatcg 420
gtcttccccc tggcaccctc ctccaagagc acctctgggg gcacagcggc cctgggctgc 480
ctggtcaagg actacttccc cgaaccggtg acggtgtcgt ggaactcagg cgccctgacc 540
agcggcgtgc acaccttccc ggctgtccta cagtcctcag gactctactc cctcagcagc 600
gtggtgaccg tgccctccag cagcttgggc acccagacct acatctgcaa cgtgaatcac 660
aagcccagca acaccaaggt ggacaagaaa gttgagccca aatcttgtga caaaactcac 720
acatgcccac cgtgcccagc acctgaactc ctggggggac cgtcagtctt cctcttcccc 780
ccaaaaccca aggacaccct catgatctcc cggacccctg aggtcacatg cgtggtggtg 840
gacgtgagcc acgaagaccc tgaggtcaag ttcaactggt acgtggacgg cgtggaggtg 900
cataatgcca agacaaagcc gcgggaggag cagtacaaca gcacgtaccg tgtggtcagc 960
gtcctcaccg tcctgcacca ggactggctg aatggcaagg agtacaagtg caaggtctcc 1020
aacaaagccc tcccagcccc catcgagaaa accatctcca aagccaaagg gcagccccga 1080
gaaccacagg tgtacaccct gcccccatcc cgggatgagc tgaccaagaa ccaggtcagc 1140
ctgacctgcc tggtcaaagg cttctatccc agcgacatcg ccgtggagtg ggagagcaat 1200
gggcagccgg agaacaacta caagaccacg cctcccgtgc tggactccga cggctccttc 1260
ttcctctaca gcaagctcac cgtggacaag agcaggtggc agcaggggaa cgtcttctca 1320
tgctccgtga tgcatgaggc tctgcacaac cactacacgc agaagagcct ctccctgtct 1380
ccgggtaaat ga 1392